# EUROPEAN PATENT SPECIFICATION

(11) **EP 1 940 694 B1**
(45) Date of publication and mention of the grant of the patent: **03.11.2010**
(21) Application number: 06790718.8
(22) Date of filing: 20.09.2006
(51) Int. Cl.: B65D 47/18, A61J 1/20, A61L 12/08, B65D 81/24, B65D 83/14, B65D 47/20

(54) **PRESSURIZED STERILE LIQUID DISPENSER**
VORRICHTUNG ZUR ABGABE VON UNTER DRUCK STEHENDER STERILER FLÜSSIGKEIT
DISTRIBUTEUR DE LIQUIDE STERILE SOUS PRESSION

(30) Priority: 20.09.2005 US 718311 P
(43) Date of publication of application: 09.07.2008
(73) Proprietor: 0736413 B.C. Ltd., Abbotsford, BC V2T 2Y8 (CA)
(72) Inventor: WEBB, Garth T., British Columbia V4A 1J7 (CA)
(74) Representative: Eisenführ, Speiser & Partner
(86) International application number: PCT/CA2006/001551
(87) International publication number: WO 2007/033480

(56) References cited:
- US-A- 4 533 068
- US-A- 4 739 906
- US-A- 5 226 568
- US-A- 5 992 701
- US-B1- 6 536 631
- US-B1- 6 896 151

## Description

### Cross-reference to Related Application

The present application claims priority from United States provisional patent application no. 60/718311 filed September 20, 2005.

### Technical Field

The invention relates to the field of dispensers for sterile liquids particularly in drops for ophthalmic purposes such as contact lens applications and eye drops, as well as nose and ear drops.

### Background

Various ophthalmic and medical applications require a hand-held dispenser of multiple doses of sterile liquids. Sterile saline is required by contact lens wearers for use as a rinse solution, for rinsing the lens prior to inserting the lens in the eye, and also for soaking the lens during the cleaning and disinfecting process. Dispensers have been designed to maintain such liquids in a sterile state for dispensing, such as disclosed in the present inventor's US Patent no. 5,507,417, which discloses a dispenser for sterile saline solution. Similarly dispensers of medicinal liquids for eye, ear and nose drops desirably keep such liquids sterile between uses to prevent bacterial growth. An example of such device is disclosed in US Patent no. 4,533,068 Meierhoefer, wherein a hydrophobic filter is used to sterilize the replacement air which enters the dispenser upon release of the squeezing pressure. Other devices use an anti-bacterial hydrophobic filter over the outlet port, or hydrophobic and hydrophilic filters in tandem, to maintain the liquid sterile. See US patent no. 3,149,758 Bush et al.; US patent no. 4,938,389 Rossi et al. and Kramer et al. US patent no. 4,463,880.

Commonly such dispensers use a one-way valve or squeeze bottle to dispense the sterile liquid. Dispensing liquid in that way can be uneven and leave wasted liquid in the container.
US 5 226 568 discloses a pressurized dispenser for sterile liquids with the features of the precharacterizing clause of claim 1. However, the document does not disclose a flexible tip expanding away from the outer surface of the plug when liquid under pressure is introduced into a space between said plug and said flexible tip to permit the liquid to be dispensed from said flexible tip and valve means being actuated by pressing said outer surface of said flexible nozzle.

There is therefore a need to avoid the foregoing problems with a pressurized sterile liquid dispenser which can be used for the delivery of sterile liquids.

### Summary of Invention

The present invention provides a reusable pressurized dispenser having a flexible dispensing nozzle that is used to dispense sterile liquid, preferably in drops. More particularly, the present invention provides a flexible nozzle for use on a pressurized canister. The pressurized canister has a storage reservoir for a pressurized sterile liquid. A plug valve or control valve permits the liquid to be dispensed from the canister by applying pressure to the flexible nozzle with the device inverted. The flexible nozzle may be fitted with a protective cap. The nozzle, valve and the protective cap may be constructed of materials which are infused with biocides to impart anti-microbial properties.

### Brief Description of Drawings

In drawings which disclose a preferred embodiment of the invention:

Fig. 1 is an isometric view in partial vertical cross section of the flexible tip and valve plug of the invention;

Fig. 2 is a front elevation of the assembly shown in Fig. 1 in partial vertical cross-section;

Fig. 3 is a rear elevation of the assembly shown in Fig. 1 in partial vertical cross-section;

Fig. 4 is a cross-section taken along lines 4-4 of Fig. 3;

Fig. 5 is a cross-section taken along lines 4-4 of Fig. 3 with valves opened;

Fig. 6 is a cross-section taken along lines 6-6 of Fig. 3;

Fig. 7 is a cross-section taken along lines 6-6 of Fig. 3 showing actuation of the flap valves;

Fig. 8 is a cross-section taken along lines 6-6 of Fig. 3 showing a second embodiment of the invention;

Fig. 9 is a cross-section taken along lines 6-6 of Fig. 3 showing a third embodiment of the invention;

Fig. 10 is an enlarged detail view of the tip in partial cross-section in closed configuration;

Fig. 11 is an enlarged detail view of the tip in partial cross-section in open configuration;

Fig. 12 is an enlarged detail view of a first further embodiment of the tip configuration;

Fig. 13 is an enlarged detail view of a second further embodiment of the tip configuration;

Fig. 14 A-G are schematic representations of alternative sealing bead cross-sections;

Fig. 15 is a cross-section taken along lines 6-6 of Fig. 3 showing a fourth embodiment of the invention;

Fig. 16 is a cross-section taken along lines 16-16 of Fig. 15;

Fig. 17 is a cross-section taken along lines 17-17 of Fig. 15 with the flap valve open position shown In phantom outline;

Fig. 18 is a front perspective view of a fifth embodiment which is not part of the present invention;

Fig. 19 is a cross-section taken along lines 19-19 of Fig. 18.

Fig. 20 is a perspective view of a sixth embodiment of the invention, taken in cross-section along lines 20-20 of Fig. 21;

Fig. 21 is a top view of the embodiment shown in Fig. 20; and

Fig. 22 is a cross-section taken along lines 20-20 of Fig. 21.

### Description

Throughout the following description, specific details are set forth in order to provide a more thorough understanding of the invention. However, the invention may be practiced without these particulars. In other instances, well known elements have not been shown or described in detail to avoid unnecessarily obscuring the invention. Accordingly, the specification and drawings are to be regarded in an illustrative, rather than a restrictive, sense.

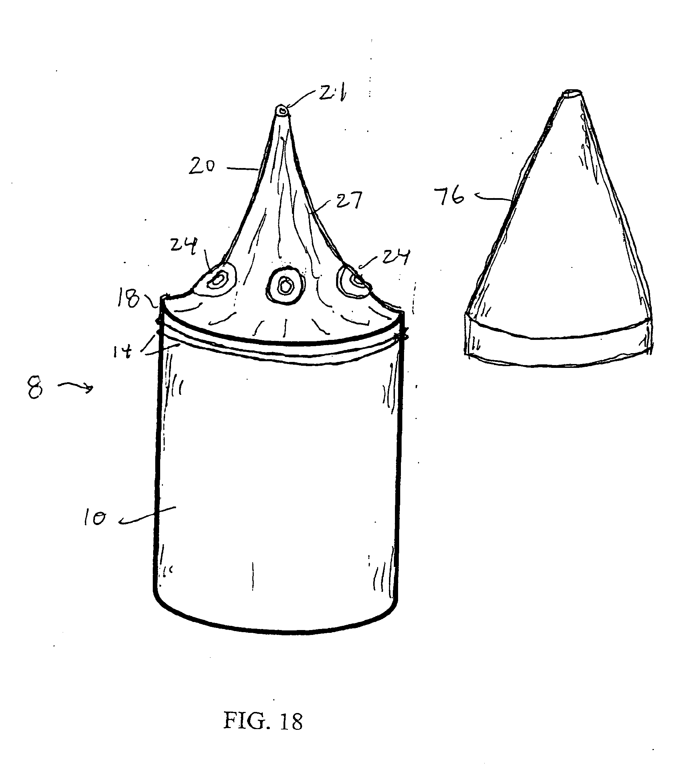

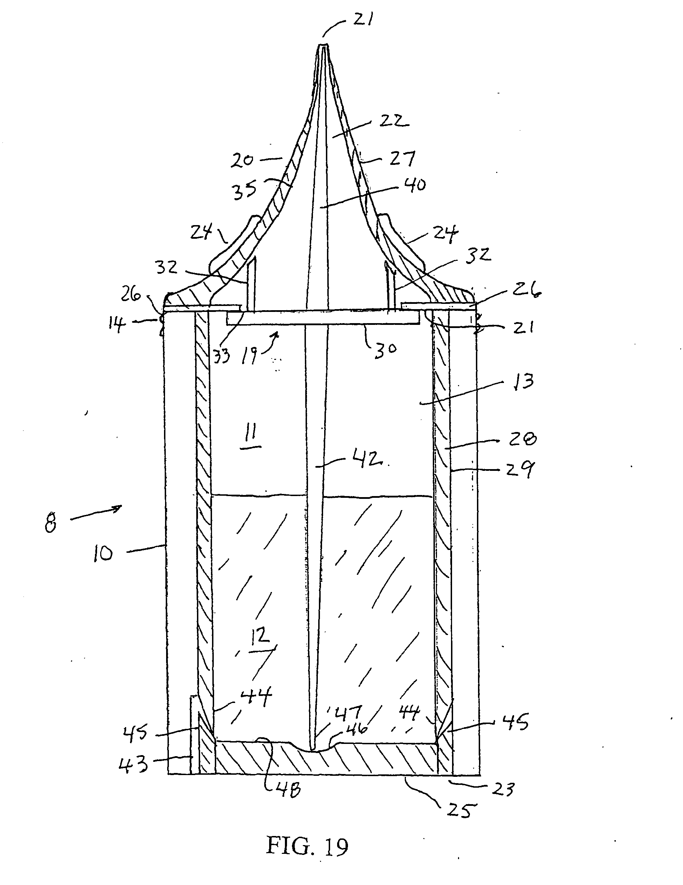

With reference first to the embodiment shown in Fig. 18 and 19 which is not part of the present invention but represents prior art useful for understanding the invention, dispenser 8 is formed of a hollow rigid cylindrical container 10 which has hollow interior 11 containing sterile liquid 12 and pressurized gas 13. Typically the container 10 will be constructed of a rigid molded plastic such as a polycarbonate. The container 10 may have a threads 14 adjacent neck 18 to which a cap 76 may be screwed in place. Alternatively cap 76 may be sized and constructed to provide a friction or snap fit over neck 18 of container 10. Container 10 has a circular open upper end 19, circular open lower end 23 and a liquid-dispensing tip 20 seated in open end 19 and having a central outlet conduit 22 communicating with outlet port 21. Circular rubber plug 25 is sealingly secured in lower open end 23.

Dispensing tip 20 is formed of flexible rubber or similar material and has conical tip section 27, actuator pads 24 and is fixed to annular flange 26 of container 10 which extends partially into opening 19 forming shoulder 21. Cylindrical rubber liner 28 which lines the interior surface 29 of container 10 and has a lower skirt 44 which acts as a continuous flap or series of flap valves in combination with flange 45 of plug 25 in connection with passage 43 described below.

A rigid plastic disc 30 has a plurality of upwardly extending posts 32 positioned inwardly of annular surface 33 of disc 30 which seats against shoulder 21, and is centrally connected to rigid control valve 40 and support element 42. In rest position, posts 32 contact the interior surface 35 of rubber dispensing tip 20 in the region of actuator pads 24, which are typically thickened sections of rubber tip 20. Also in rest position the upper end of control valve 40 extends into and seals outlet port 21.

In rest position, disc 30 is held in place seated against shoulder 21 by the pressure inside container 10. Support element 42, which has a lower tapered end 47 which extends into and bears against depression 46 in the inner surface 48 of rubber plug 25 in order to keep disc 30 centered. Plug 25 has circular flange 45 which sealingly fits against the inner surface 29 of container 10. One or more passages 43 extend along the inner surface 29 of container 10. Passages 43 are used to inject the pressurized gas 13 into the interior 11 of container 10.

Typically a pharmacist will purchase empty dispensers 8 and fill them with sterile liquid under pressure. To fill the device with sterilized liquid, the pharmacist inverts the capped dispenser 8, removes plug 25, fills with distilled water, replaces plug 25 and injects an inert pressurized gas through passage 43. The unit 8 is then irradiated for sterility and packaged for sale to the consumer.

To operate the device, after purchase the user turns container 10 upside down, and presses actuator pads 24. That forces posts 32 and attached circular disc 30 off its seat 33 against shoulder 21 of rubber tip 20 and removes the end of actuator valve 40 from outlet port 21, opening outlet port 21. The pressure in the interior 11 of container 10 then forces the sterile liquid 12 around disc 30 and out through outlet port 21. The diameter and flexibility of port 21 are selected so that droplets of suitable size are formed within the selected time frame. When pressure is removed from actuator pads 24, the disc 30 returns to its sealing position against shoulder 21 due to the pressure in interior 11. Support post 42 serves to center disc 30 in the open end 19.

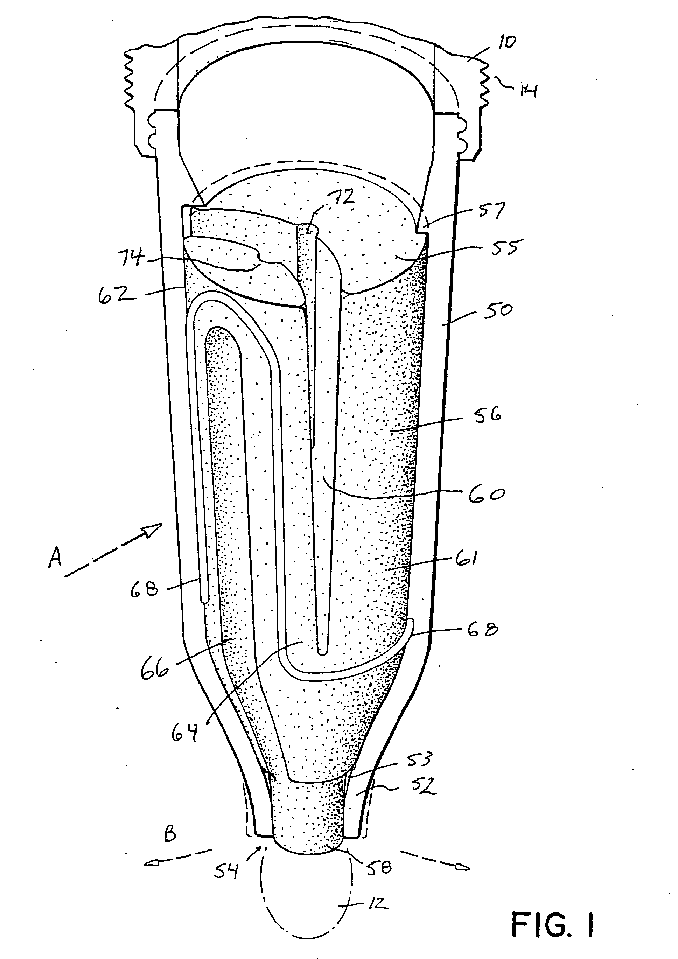

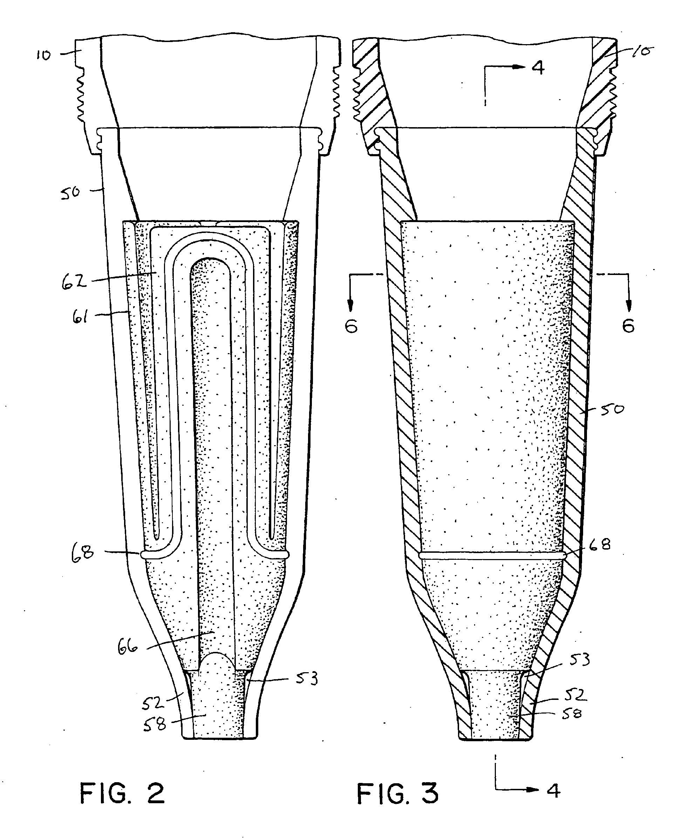

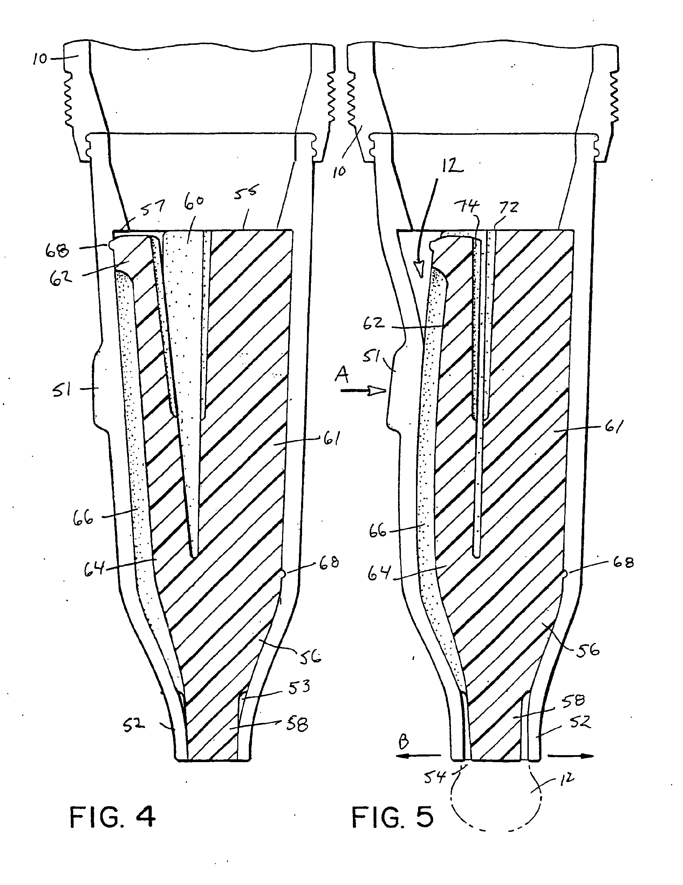

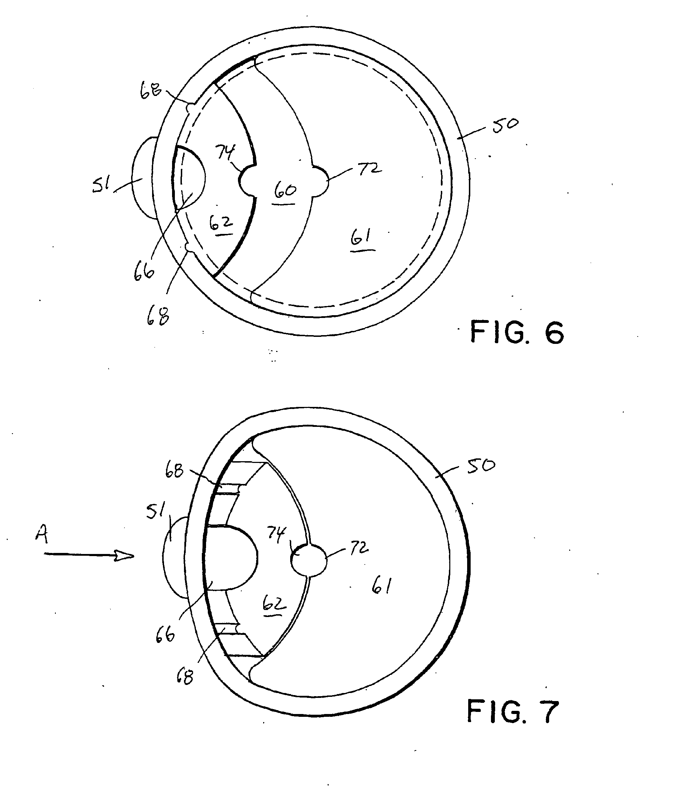

Figures 1 through 7 illustrate a second embodiment of the flexible tip 20 which may be used in the invention. Flexible dispensing nozzle 50 formed of flexible rubber or similar material has conical tip section 52 with dispensing opening 54 and actuator pad 51. A plug valve 56 has a cylindrical tip 58 which extends into and seals opening 54 when the device is at rest. The upper end 55 of plug valve 56 seats against shoulder 57 of nozzle 50. Plug valve 56 is constructed of plastic or similar material and has a central slot 60 which separates the rigid body 61 of plug valve 56 from a hinged valve flap 62, which is able to flex in area 64 and has a lengthwise groove 66. A raised sealing bead 68 surrounds groove 66. Central channels 72, 74 may be provided in body 61 and flap 62 to assist in returning valve flap 62 to its rest position as described further below.

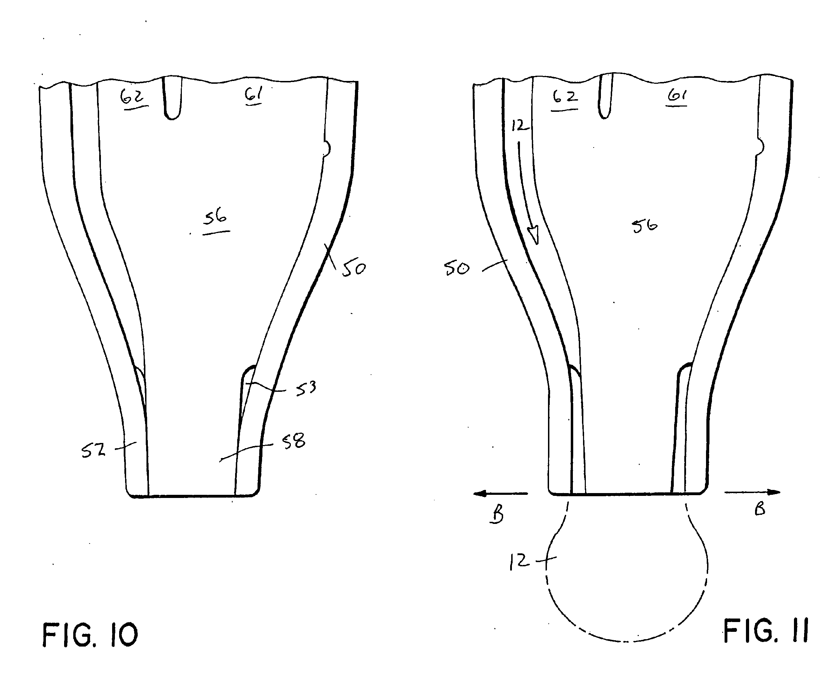

When at rest the flexible nozzle 50, which has a diameter at rest which is slightly less than the exterior diameter of plug valve 56, seals to the exterior of plug valve 56 and no liquid can exit from opening 54. When a user squeezes the flexible nozzle between thumb and index finger in the region of actuator pad 51, in the direction shown by arrow A, the free end of valve flap 62 bends away from the flexible nozzle 50, allowing pressurized liquid 12 to flow out of canister 10 and down groove 66 into space 53. The pressure of the liquid in space 53 causes tip section 52 to open away from cylindrical tip 58 in the direction shown by arrows B and liquid 12 flows out opening 54. See Fig. 10 and 11. The diameter and flexibility of opening 54, space 53 and the size of tip 58 are selected so that droplets of liquid 12 of suitable size are formed within the selected time frame. When pressure from the user's fingers is released, the valve flap 62 returns to its rest position, assisted by the pressure of liquid in channels 72, 74, and sealing bead 68 of plug valve 56 seals against the flexible nozzle 50 once again and flow of liquid 12 ceases.

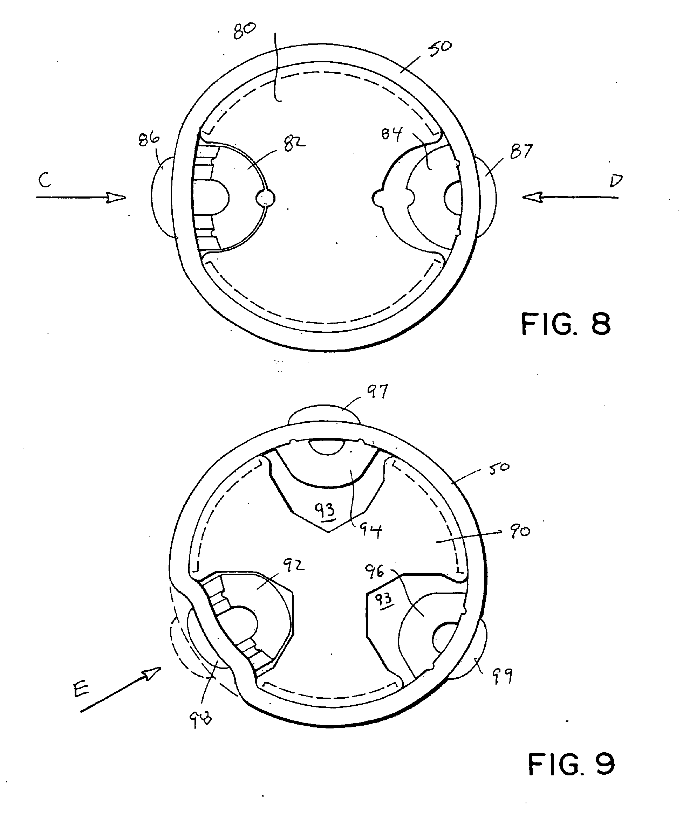

Fig. 8 illustrates a variant of the embodiment of the invention shown in Fig. 1-7 in which plug valve 80 has two flap valves 82, 84, each having the construction as described above. Flexible nozzle 50 may have two actuator pads 86, 87 so that application of pressure to one or both pads 86, 87 in the direction shown by arrows C, D will open a passage for delivery of the pressurized liquid 12 as described above. Fig. 9 illustrates a variant of the embodiment of the invention shown in Fig. 1-7 in which plug valve 90 has three flap valves 92, 94, 96 each having the construction as described above except that in this case the channels 72, 74 are replaced with an angular passage 93. Flexible nozzle 50 in this case may have three actuator pads 97, 98, 99 so that application of pressure to one of the pads as shown by arrow E will open a passage for delivery of the pressurized liquid 12 as described above.

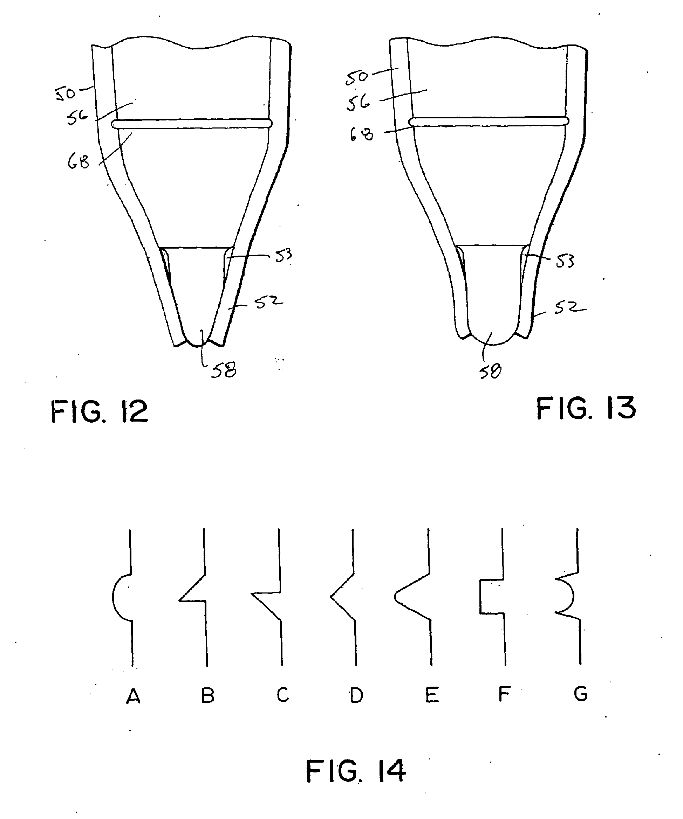

Figures 12 and 13 illustrate alternative shapes for the tip 58 of plug valve 56, being tapered with a rounded end as in Fig. 12 or cylindrical with a hemispherical end as in Fig. 13. Fig. 14 illustrates different cross-sectional shapes which may be suitable for sealing bead 68.

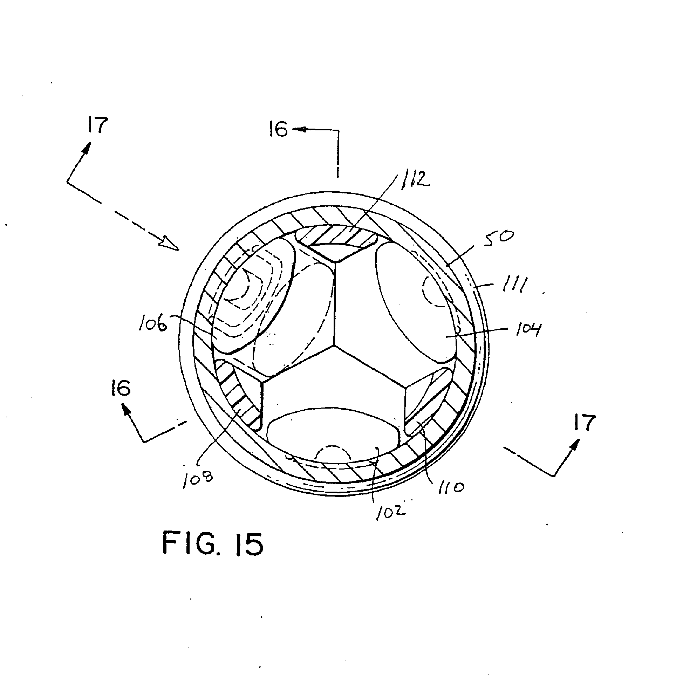

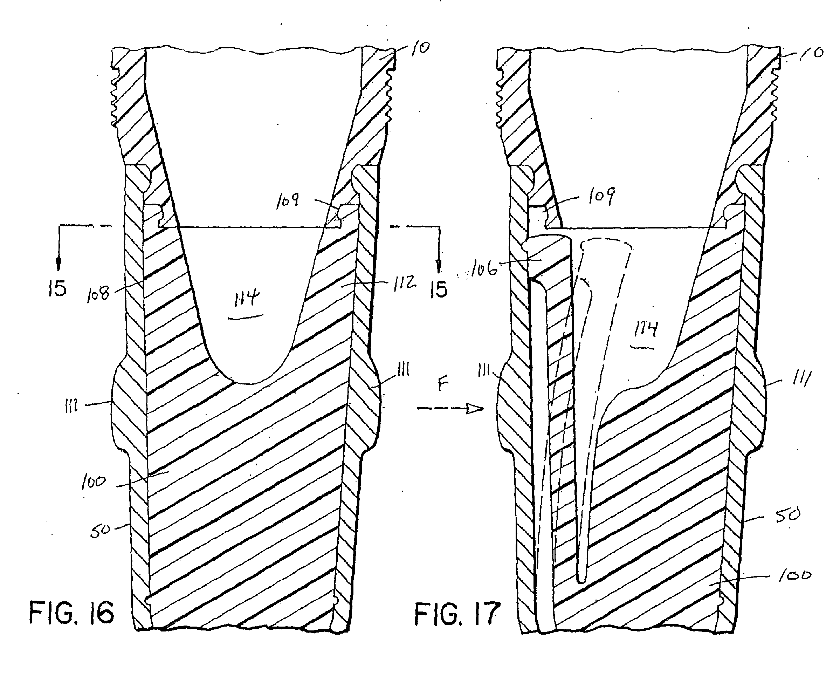

Figures 15 through 17 illustrate a further embodiment of the invention in which the plug valve 100 has three flap valves 102, 104, 106, each having generally the same construction as the embodiment in Fig. 9 but permitting greater movement of the flap valves. Plug valve 100 is connected directly to canister 10 by three upstanding posts 108, 110, 112 which snap fit into groove 109 and form a central cavity 114. Pressing circumferential actuator pad 111 as illustrated by arrow F will open a passage for delivery of the pressurized liquid 12 as described above.

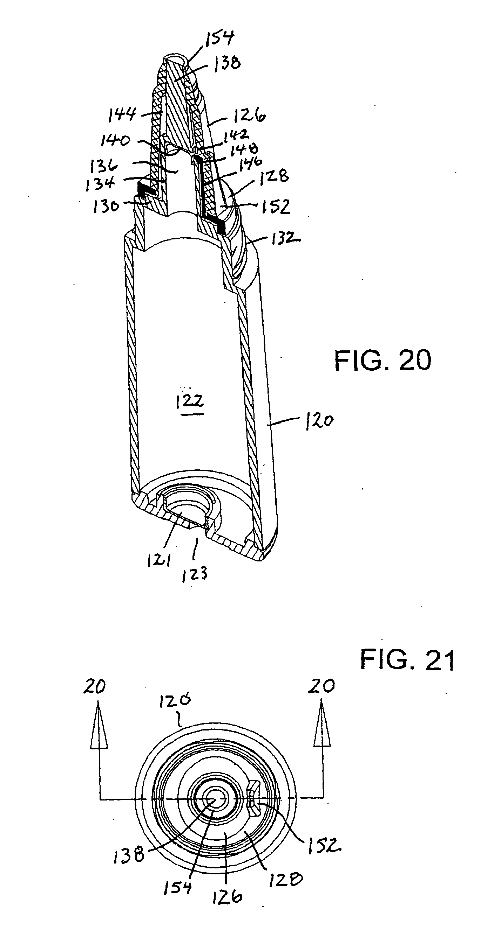

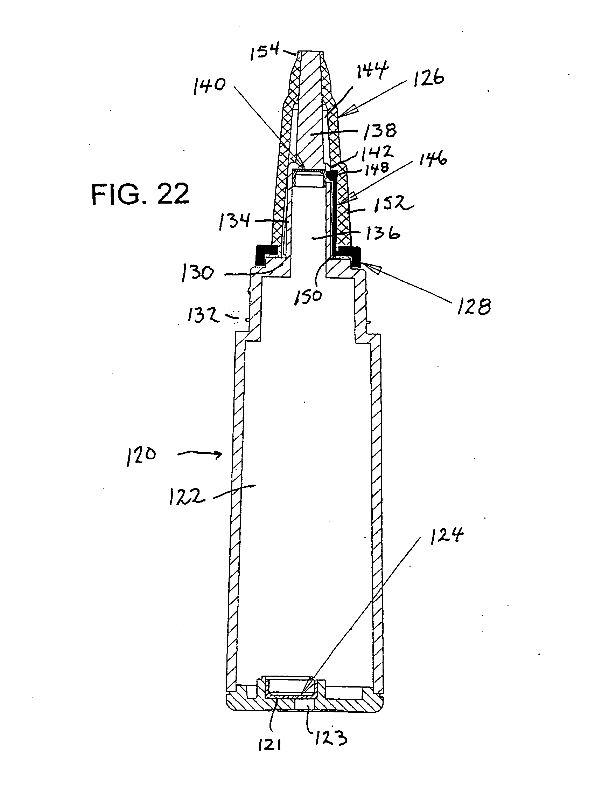

Figures 20 through 22 illustrate a further embodiment of the invention. The pressurized canister 120 has pressurized storage reservoir 122 for storing the sterile liquid (not shown), which is injected into the reservoir through passage 123 and bottom filling valve 124. Bottom filling valve 124 is sealed by a cylindrical elastomeric valve 121 which releases away from passage 123 when liquid or gas under pressure is introduced into passage 123. Flexible nozzle 126 is molded of a rubber such as used in syringes and is molded or welded integrally onto a rigid retainer ring 128. Retainer ring 128 and attached nozzle 126 are secured by glue or welding to neck 130 of canister 120, which has threads 132 for attachment of a cap. Extending upwardly from neck 130 is hollow cylindrical element 134 which surrounds cylindrical chamber 136 and which connects at its upper end to solid conical plug 138. A hollow cylindrical elastomeric valve 140 is seated against the lower surface of plug 138, at the upper end of chamber 136. At rest, valve 140 seals a narrow slot 142 which communicates between chamber 136 and annular chamber 144 between the outer surface of plug 138 and the inner surface of nozzle 126 which extends over plug 138. A matchstick-shaped actuator 146 is hingedly connected to retaining ring 128 at 150 and has a knob 148 which touches the outer surface of valve 140 and moves valve 140 out of slot 142 when actuating button 152 is pressed, thereby pivoting knob 148 inwardly against valve 140.

To operate the embodiment shown in Fig. 20, the device is inverted and actuating button 152 is pressed, thereby pivoting knob 148 inwardly against valve 140, opening slot 142. When slot 142 is opened, liquid under pressure from reservoir 122 is forced through slot 142 into chamber 144 and moves tip 154 away from plug 138 to form a liquid droplet at the end of nozzle 126. The size of slot 142, chamber 144 and the diameter and flexibility of tip 154 are selected so that droplets of liquid 12 of suitable size are formed within the selected time frame. Once the droplet releases, the actuator button 152 is released. An exemplary diameter of slot 142 is 2 mm. , where matchstick actuator 146 has a width which is slightly less than slot 142. Also, the outer surface of plug 138 can be provided with lengthwise or annular grooves to regulate the speed of accumulation of drops.

In the embodiment shown in Fig. 20, the cylindrical chamber on the down stream side of the valve 140 is formed to hold some of the liquid contents of the storage chamber 122 in place by capillary force even when the unit is held in the upright position. This feature is useful to prevent the gaseous propellant from escaping inadvertently if the valve 140 is actuated while in the upright position. When in the upright position, preferably valve 140 is sized so that capillary force keeps a small volume of liquid in the area just below the valve. In this way if the actuator 152 is accidentally pushed in the upright position only a small amount of liquid will be expelled rather than all the pressurized gas. To achieve this the valve 140 preferably has a diameter between 2 and 14 mm, depending on the viscosity of liquid 12.

A pressure relief valve may be installed on the down-stream side of the control valve to act as an additional protective barrier to contaminants. Additionally or alternatively, a protective cap containing a retaining element with biocide within it may be used to provide anti-microbial protection to the outlet port of the nozzle.

The construction materials used to fabricate the components of the invention as described herein may be infused with biocides, such as metallic releasing agents, to control the growth of organisms on and around the dispensing port in order to limit the contamination of the liquid as it passes out of the storage reservoir. The inner surface of the flexible nozzle and/or the tip of the control valve or plug valve may also be lined with metallic silver. Alternatively, a lid possessing a retaining element to hold a biocide against the exit port may be used to limit the contamination of the liquid.

Cap 76 may be a disinfectant cap as disclosed in co-pending patent application PCT/2005/001094, filed July 13, 2005. In that case, cap 76 may be provided with a biocide-containing pad fixed to the upper inner surface of cap 76 so that after liquid is dispensed and cap 76 is replaced, the biocide on the cap will contact and disinfect the outlet port 21.

As will be apparent to those skilled in the art in the light of the foregoing disclosure, many alterations and modifications are possible in the practice of this invention without departing from the scope thereof. While embodiments having one, two or three flap valves have been illustrated, embodiments having four or more flap valves of the same structure, with appropriate modification to the size of the flexible nozzle are also within the scope of the invention.

## Claims

1. A pressurized dispenser for sterile liquids, comprising:
a) a storage chamber (122) for containing a pressurized sterile liquid;
b) a flexible dispensing nozzle (50, 126) comprising an outer surface and communicating with said storage chamber (122) and comprising a flexible fluid-dispensing tip comprising inner and outer sides;
c) a plug (25, 138) extending into said flexible tip (20) whereby said flexible tip (20) sealingly contacts an outer surface of said plug (25, 138) when pressure is equal on the inner and outer sides of the flexible tip (20);
d) an intermediate chamber (136) communicating with the space between said plug (25, 138) and said flexible tip (20) and alternately communicating with and sealed from said storage chamber (122); and
e) valve means (40, 140) actuable to control flow of said pressurized sterile liquid from said storage chamber (122) to said intermediate chamber (136) to thereby selectively introduce liquid under pressure into the space between said plug (25, 138) and said flexible tip (20) and cause said flexible tip (20) to expand away from said outer surface of said plug (25, 138) to permit the liquid to be dispensed from said flexible tip (20);
**characterized in that** said tip (20) radially expands away from said outer surface of said plug (25, 138) when liquid under pressure is introduced into a space between said plug (25, 138) and said flexible tip (20), and wherein said valve means (40, 140) is actuated by pressing said outer surface of said flexible nozzle (50, 126).

2. The pressurized dispenser of claim 1 wherein said plug (25,138) is generally cylindrical.

3. The pressurized dispenser of claim 1 wherein said valve (40, 140) comprises a hinged element (62, 146) biased to a position to sealingly close the communication between said storage chamber (122) and said intermediate chamber (136) and movable between said sealing position and a position which allows flow of said pressurized liquid into said intermediate chamber (136) by pressing said outer surface of said flexible nozzle (50, 126).

4. The pressurized dispenser of claim 1 wherein said valve (40,140) comprises two hinged elements (62, 146) each biased to a position to sealingly close the communication between said storage chamber (122) and said intermediate chamber (136) and movable between said sealing position and a position which mallows flow of said pressurized liquid into said intermediate chamber (136) by pressing said outer surface of said flexible nozzle (50, 126).

5. The pressurized dispenser of claim 1 wherein said valve (40, 140) comprises three hinged elements (62,146) each biased to a position to sealingly close the communication between said storage chamber (122) and said intermediate chamber (136) and movable between said sealing position and a position which allows flow of said pressurized liquid into said intermediate chamber (136) by pressing said surface of said flexible nozzle (50, 126).

6. The pressurized dispenser of claim 1 wherein said valve (40,140) comprises a disc (30) biased to a position to sealingly close the communication between said storage chamber (122) and said intermediate chamber (136) and movable between said sealing position and a position which allows flow of said pressurized liquid into said intermediate chamber (136) by pressing said outer surface of said flexible nozzle (50, 126).

7. The pressurized dispenser of claim 1 wherein said valve (40, 140) comprises an elastomeric element biased to a position to sealingly close the communication between said storage chamber (122) and said intermediate chamber (136) and movable between said sealing position and a position which allows flow of said pressurized liquid into said intermediate chamber (136) by pressing said outer surface of said flexible nozzle (50, 126).

8. The pressurized dispenser of claim 7 wherein said elastomeric element is located in a valve chamber communicating between said storage chamber (122) and said intermediate chamber (136) and said valve chamber is sized to retain liquid by capillary force when said dispenser is upright.

9. The pressurized dispenser of claim 1 wherein the size of said intermediate chamber (136) and the diameter and flexibility of said flexible tip (20) are selected so that droplets of liquid of a pre-determined size are formed within a pre-determined time.

10. The pressurized dispenser of claim 1 comprising a passage which communicates between said intermediate chamber (136) and the space between said plug (25, 138) and said flexible tip (20) and wherein the size of said passage, said intermediate chamber (136) and the diameter and flexibility of said flexible tip (20) are selected so that droplets of liquid of a pre-determined size are formed within a pre-determined time.

11. The pressurized dispenser of claim 1 further comprising a protective cap (76).

12. The pressurized dispenser of claim 1 wherein said flexible nozzle (50, 126) is constructed of materials which are infused with biocides to impart anti-microbial properties.

13. The pressurized dispenser of claim 1 wherein said flexible nozzle (50, 126) and valve (40, 140) are constructed of materials which are infused with biocides to impart anti-microbial properties.

14. The pressurized dispenser of claim 11 wherein said flexible nozzle (50, 126), valve (40, 140) and protective cap (76) are constructed of materials which are infused with biocides to impart anti-microbial properties.

15. A method of dispensing drops of sterile liquids, comprising:
a) providing: i) storage chamber (122) for containing a pressurized sterile liquid; ii) a flexible dispensing nozzle (50, 1216) comprising an outer surface and communicating with said storage chamber (122) and comprising a flexible fluid-dispensing tip (20); iii) a plug (25, 138) extending into said flexible tip (20) comprising inner and outer sides whereby said flexible tip (20) sealingly contacts an outer surface of said plug (25, 138) when pressure is equal on the inner and outer sides of the flexible tip (20), and radially expands away from said outer surface of said plug (25, 138) when liquid under pressure is introduced into a space between said plug (25, 138) and said flexible tip (20); iv) an intermediate chamber (136) communicating with said space between said plug (25, 138) and said flexible tip (20) and alternately communicating with and sealed from said storage chamber (122); and v) valve means (40, 140) actuable to control flow of said pressurized sterile liquid from said storage chamber (122) to said intermediate chamber (136) to thereby selectively introduce liquid under pressure into the space between said plug (25, 138) and said flexible tip (20) and cause said flexible tip (20) to expand away from said outer surface of said plug (25, 138) to permit the liquid to be dispensed from said flexible tip (20);
b) providing a pressurized sterile liquid in said storage chamber (122);
c) inverting said dispenser;
d) pressing said outer surface of said flexible nozzle (50,126) to thereby actuate said valve means (40,140)and permit the sterile liquid to be dispensed from said flexible tip (20) as one or more drops; and
e) releasing pressure from the surface of said flexible nozzle (50, 126) when the desired volume of liquid has been dispensed.

## Patentansprüche

1. Unter Druck stehender Dispenser für sterile Flüssigkeiten, umfassend:
a) eine Aufbewahrungskammer (122) zum Enthalten einer unter Druck stehenden sterilen Flüssigkeit;
b) eine flexible Dispensier-Düse (50, 126), die eine äußere Oberfläche umfasst und mit der Aufbewahrungskammer (122) in Verbindung steht und eine flexible Flüssigkeit-dispensierende Spitze umfasst, die Innen- und Außenseiten umfasst;
c) einen Stopfen (25, 138), der in die flexible Spitze (20) reicht, wodurch die flexible Spitze (20) eine äußere Oberfläche von dem Stopfen (25, 138) abdichtend kontaktiert, wenn an den Innen- und Außenseiten der flexiblen Spitze (20) gleicher Druck anliegt;
d) eine Zwischenkammer (136), die mit dem Raum zwischen dem Stopfen (25, 138) und der flexiblen Spitze (20) in Verbindung steht und die alternierend in Verbindung steht mit und abgedichtet ist von der Aufbewahrungskammer (122); und
e) Ventileinrichtung (40, 140), betätigbar zum Kontrollieren eines Flusses der unter Druck stehenden sterilen Flüssigkeit von der Aufbewahrungskammer (122) zu der Zwischenkammer (136), um **dadurch** selektiv unter Druck stehende Flüssigkeit in den Raum zwischen dem Stopfen (25, 138) und der flexiblen Spitze (20) einzuführen und um hervorzurufen, dass die flexible Spitze (20) von der äußeren Oberfläche des Stopfens (25, 138) wegexpandiert, um zuzulassen, dass die Flüssigkeit von der flexiblen Spitze (20) dispensiert wird;
**dadurch gekennzeichnet, dass** die Spitze (20) radial von der äußeren Oberfläche des Stopfens (25, 138) wegexpandiert, wenn unter Druck stehende Flüssigkeit in einen Raum zwischen dem Stopfen (25, 138) und der flexiblen Spitze (20) eingeführt wird, und wobei die Ventileinrichtung (40, 140) durch Pressen der äußeren Oberfläche von der flexiblen Düse (50, 126) betätigt wird.

2. Unter Druck stehender Dispenser gemäß Anspruch 1, wobei der Stopfen (25, 138) allgemein zylindrisch ist.

3. Unter Druck stehender Dispenser gemäß Anspruch 1, wobei das Ventil (40, 140) ein klappbares Element (62, 146) umfasst, das in einer Position voreingestellt ist, um die Verbindung zwischen der Aufbewahrungskammer (122) und der Zwischenkammer (136) abdichtend zu schließen, und das beweglich ist zwischen der Abdichtungsposition und einer Position, die einen Fluss von der unter Druck stehender Flüssigkeit in die Zwischenkammer (136) durch Pressen der äußeren Oberfläche von der flexiblen Düse (50, 126) erlaubt.

4. Unter Druck stehender Dispenser gemäß Anspruch 1, wobei das Ventil (40, 140) zwei klappbare Elemente (62, 146) umfasst, wobei jede in einer Position voreingestellt ist, um die Verbindung zwischen der Aufbewahrungskammer (122) und der Zwischenkammer (136) abdichtend zu schließen, und die beweglich sind zwischen der Abdichtungsposition und einer Position, die einen Fluss von der unter Druck stehenden Flüssigkeit in die Zwischenkammer (136) durch Pressen der äußeren Oberfläche von der flexiblen Düse (50, 126) erlaubt.

5. Unter Druck stehender Dispenser gemäß Anspruch 1, wobei das Ventil (40, 140) drei klappbare Elemente (62, 146) umfasst, wobei jede in einer Position voreingestellt ist, um die Verbindung zwischen der Aufbewahrungskammer (122) und der Zwischenkammer (136) abdichtend zu schließen, und die beweglich sind zwischen der Abdichtungsposition und einer Position, die einen Fluss von der unter Druck stehenden Flüssigkeit in die Zwischenkammer (136) durch Pressen der äußeren Oberfläche von der flexiblen Düse (50, 126) erlaubt.

6. Unter Druck stehender Dispenser gemäß Anspruch 1, wobei das Ventil (40, 140) eine Scheibe (30) umfasst, die in einer Position voreingestellt ist, um die Verbindung zwischen der Aufbewahrungskammer (122) und der Zwischenkammer (136) abdichtend zu schließen, und die beweglich ist zwischen der Abdichtungsposition und einer Position, die einen Fluss von der unter Druck stehenden Flüssigkeit in die Zwischenkammer (136) durch Pressen der äußeren Oberfläche von der flexiblen Düse (50, 126) erlaubt.

7. Unter Druck stehender Dispenser gemäß Anspruch 1, wobei das Ventil (40, 140) ein elastomeres Element umfasst, das in einer Position voreingestellt ist, um die Verbindung zwischen der Aufbewahrungskammer (122) und der Zwischenkammer (136) abdichtend zu schließen, und die beweglich ist zwischen der Abdichtungsposition und einer Position, die einen Fluss von der unter Druck stehenden Flüssigkeit in die Zwischenkammer (136) durch Pressen der äußeren Oberfläche von der flexiblen Düse (50, 126) erlaubt.

8. Unter Druck stehender Dispenser gemäß Anspruch 7, wobei das elastomere Element in einer Ventilkammer angeordnet ist, die die Aufbewahrungskammer (122) und die Zwischenkammer (136) verbindet, und wobei die Ventilkammer bemessen ist, um Flüssigkeit durch Kapillarkraft zurückzuhalten, wenn die Vorrichtung aufrecht ist.

9. Unter Druck stehender Dispenser gemäß Anspruch 1, wobei die Größe der Zwischenkammer (136) und der Durchmesser und die Flexibilität von der flexiblen Spitze (20) so ausgewählt sind, dass Flüssigkeitstropfen in einer vorbestimmten Größe innerhalb einer vorbestimmten Zeit gebildet werden.

10. Unter Druck stehender Dispenser gemäß Anspruch 1, umfassend eine Passage, die die Zwischenkammer (136) und den Raum zwischen dem Stopfen (25, 138) und der flexiblen Spitze (20) verbindet, und wobei die Größe von der Passage, der Zwischenkammer (136) und der Durchmesser und die Flexibilität von der flexiblen Spitze (20) so ausgewählt sind, dass Flüssigkeitstropfen einer vorbestimmten Größe innerhalb einer vorbestimmten Zeit gebildet werden.

11. Unter Druck stehender Dispenser gemäß Anspruch 1, weiter umfassend eine schützende Kappe (76).

12. Unter Druck stehender Dispenser gemäß Anspruch 1, wobei die flexible Düse (50, 126) aus Materialien konstruiert ist, die mit Bioziden durchdrungen sind, um antimikrobielle Eigenschaften zu verleihen.

13. Unter Druck stehender Dispenser gemäß Anspruch 1, wobei die flexible Düse (50, 126) und das Ventil (40, 140) aus Materialien konstruiert sind, die mit Bioziden durchdrungen sind, um antimikrobielle Eigenschaften zu verleihen.

14. Unter Druck stehender Dispenser gemäß Anspruch 11, wobei die flexible Düse (50, 126), das Ventil (40, 140) und die schützende Kappe (76) aus Materialien konstruiert sind, die mit Bioziden durchdrungen sind, um antimikrobielle Eigenschaften zu verleihen.

15. Verfahren zum Dispensieren von Tropfen steriler Flüssigkeiten, umfassend:
a) Bereitstellen von:
i) einer Aufbewahrungskammer (122) zum Enthalten einer unter Druck stehenden sterilen Flüssigkeit; ii) einer flexiblen Dispensier-Düse (50, 1216), die eine äußere Oberfläche umfasst, und die mit der Aufbewahrungskammer (122) verbunden ist, und die eine flexible Flüssigkeit-dispensierende Spitze (20) umfasst; iii) einem Stopfen (25, 138), der in die flexible Spitze (20) reicht, die Innen- und Außenseiten umfasst, wodurch die flexible Spitze (20) eine äußere Oberfläche des Stopfens (25, 138) abdichtend kontaktiert, wenn an den Innen- und Außenseiten von der flexiblen Spitze (20) gleicher Druck anliegt, und die radial von der äußeren Oberfläche von dem Stopfen (25, 138) wegexpandiert, wenn unter Druck stehende Flüssigkeit in einen Raum zwischen dem Stopfen (25, 138) und der flexiblen Spitze (20) eingeführt wird; iv) einer Zwischenkammer (136), die mit dem Raum zwischen dem Stopfen (25, 138) und der flexiblen Spitze (20) in Verbindung steht, und die alternierend in Verbindung steht mit und abgedichtet ist von der Aufbewahrungskammer (122); und v) einer Ventileinrichtung (40, 140), betätigbar zum Kontrollieren eines Flusses von der unter Druck stehenden sterilen Flüssigkeit von der Aufbewahrungskammer (122) zu der Zwischenkammer (136), um **dadurch** selektiv unter Druck stehende Flüssigkeit in den Raum zwischen dem Stopfen (25, 138) und der flexiblen Spitze (20) einzuführen und um hervorzurufen, dass die flexible Spitze (20) von der äußeren Oberfläche von dem Stopfen (25, 138) wegexpandiert, um zuzulassen, dass die Flüssigkeit von der flexiblen Spitze (20) dispensiert wird;
b) Bereitstellen einer unter Druck stehenden sterilen Flüssigkeit in der Aufbewahrungskammer (122);
c) Umkehren des Dispensers;
d) Pressen der äußeren Oberfläche der flexiblen Düse (50, 126), um **dadurch** die Ventileinrichtung (40, 140) zu betätigen, und um zuzulassen, dass die sterile Flüssigkeit von der flexiblen Spitze (20) als eine oder mehrere Tropfen dispensiert wird; und
e) Lösen von Druck von der Oberfläche der flexiblen Düse (50, 126), wenn das gewünschte Flüssigkeitsvolumen dispensiert worden ist.

## Revendications

1. Distributeur sous pression pour des liquides stériles, comprenant :
a) une chambre de stockage (122) pour contenir un liquide stérile sous pression ;
b) une buse de distribution flexible (50, 126) comprenant une surface extérieure et communiquant avec ladite chambre de stockage (122) et comprenant un embout flexible de distribution de fluide comprenant des côtés intérieur et extérieur ;
c) un obturateur (25, 138) s'étendant dans ledit embout flexible (20), moyennant quoi ledit embout flexible (20) entre en contact hermétique avec une surface extérieure dudit obturateur (25, 138) lorsqu'une pression est égale sur les côtés intérieur et extérieur de l'embout flexible (20) ;
d) une chambre intermédiaire (136) communiquant avec l'espace entre ledit obturateur (25, 138) et ledit embout flexible (20) et communiquant en alternance avec et isolée de façon hermétique de ladite chambre de stockage (122) ; et
e) des moyens de soupape (40, 140) actionnables pour réguler l'écoulement dudit liquide stérile sous pression de ladite chambre de stockage (122) à ladite chambre intermédiaire (136) pour ainsi introduire sélectivement le liquide sous pression dans l'espace entre ledit obturateur (25, 138) et ledit embout flexible (20) et faire en sorte que ledit embout flexible (20) s'agrandisse pour s'éloigner de ladite surface extérieure dudit obturateur (25, 138) pour permettre au liquide d'être distribué à partir dudit embout flexible (20) ;
**caractérisé en ce que** ledit embout (20) s'agrandit de façon radiale pour s'éloigner de ladite surface extérieure dudit obturateur (25, 138) lorsque le liquide sous pression est introduit dans un espace entre ledit obturateur (25, 138) et ledit embout flexible (20), et dans lequel lesdits moyens de soupape (40, 140) sont actionnés en appuyant sur ladite surface extérieure de ladite buse flexible (50, 126).

2. Distributeur sous pression selon la revendication 1, dans lequel ledit obturateur (25, 138) est généralement cylindrique.

3. Distributeur sous pression selon la revendication 1, dans lequel ladite soupape (40, 140) comprend un élément articulé (62, 146) sollicité jusqu'à une position pour fermer, de façon hermétique, la communication entre ladite chambre de stockage (122) et ladite chambre intermédiaire (136) et mobile entre ladite position de fermeture hermétique et une position qui permet l'écoulement dudit liquide sous pression dans ladite chambre intermédiaire (136) en appuyant sur ladite surface extérieure de ladite buse flexible (50, 126).

4. Distributeur sous pression selon la revendication 1, dans lequel ladite soupape (40, 140) comprend deux éléments articulés (62, 146) chacun sollicités jusqu'à une position pour fermer, de façon hermétique, la communication entre ladite chambre de stockage (122) et ladite chambre intermédiaire (136) et mobile entre ladite position de fermeture hermétique et une position qui permet l'écoulement dudit liquide sous pression dans ladite chambre intermédiaire (136) en appuyant sur ladite surface extérieure de ladite buse flexible (50, 126).

5. Distributeur sous pression selon la revendication 1, dans lequel ladite soupape (40, 140) comprend trois éléments articulés (62, 146) chacun sollicités jusqu'à une position pour fermer, de façon hermétique, la communication entre ladite chambre de stockage (122) et ladite chambre intermédiaire (136) et mobile entre ladite position de fermeture hermétique et une position qui permet l'écoulement dudit liquide sous pression dans ladite chambre intermédiaire (136) en appuyant sur ladite surface de ladite buse flexible (50, 126).

6. Distributeur sous pression selon la revendication 1, dans lequel ladite soupape (40, 140) comprend un disque (30) sollicité jusqu'à une position pour fermer, de façon hermétique, la communication entre ladite chambre de stockage (122) et ladite chambre intermédiaire (136) et mobile entre ladite position de fermeture hermétique et une position qui permet l'écoulement dudit liquide sous pression dans ladite chambre intermédiaire (136) en appuyant sur ladite surface extérieure de ladite buse flexible (50, 126).

7. Distributeur sous pression selon la revendication 1, dans lequel ladite soupape (40, 140) comprend un élément élastomère sollicité jusqu'à une position pour fermer, de façon hermétique, la communication entre ladite chambre de stockage (122) et ladite chambre intermédiaire (136) et mobile entre ladite position de fermeture hermétique et une position qui permet l'écoulement dudit liquide sous pression dans ladite chambre intermédiaire (136) en appuyant sur ladite surface extérieure de ladite buse flexible (50, 126).

8. Distributeur sous pression selon la revendication 7, dans lequel ledit élément élastomère est situé dans une chambre de soupape communiquant entre ladite chambre de stockage (122) et ladite chambre intermédiaire (136) et ladite chambre de soupape est dimensionnée pour retenir le liquide par force capillaire lorsque ledit distributeur est vertical.

9. Distributeur sous pression selon la revendication 1, dans lequel la taille de ladite chambre intermédiaire (136) et le diamètre et la flexibilité dudit embout flexible (20) sont sélectionnés de sorte que des gouttelettes de liquide d'une taille prédéterminée soient formées au sein d'une période prédéterminée.

10. Distributeur sous pression selon la revendication 1, comprenant un passage qui communique entre ladite chambre intermédiaire (136) et l'espace entre ledit obturateur (25, 138) et ledit embout flexible (20) et dans lequel la taille dudit passage, de ladite chambre intermédiaire (136) et le diamètre et la flexibilité dudit embout flexible (20) sont sélectionnés de sorte que des gouttelettes de liquide d'une taille prédéterminée soient formées au sein d'une période prédéterminée.

11. Distributeur sous pression selon la revendication 1, comprenant en outre un capuchon de protection (76).

12. Distributeur sous pression selon la revendication 1, dans lequel ladite buse flexible (50, 126) est faite de matériaux qui sont imprégnés de biocides pour fournir des propriétés antimicrobiennes.

13. Distributeur sous pression selon la revendication 1, dans lequel ladite buse flexible (50, 126) et ladite soupape (40, 140) sont faites de matériaux qui sont imprégnés de biocides pour fournir des propriétés antimicrobiennes.

14. Distributeur sous pression selon la revendication 11, dans lequel ladite buse flexible (50, 126), ladite soupape (40, 140) et ledit capuchon de protection (76) sont faits de matériaux qui sont imprégnés de biocides pour fournir des propriétés antimicrobiennes.

15. Procédé de distribution de gouttes de liquides stériles, comprenant les étapes consistant à :
a) fournir : i) une chambre de stockage (122) pour contenir un liquide stérile sous pression ; ii) une buse de distribution flexible (50, 1216) comprenant une surface extérieure et communiquant avec ladite chambre de stockage (122) et comprenant un embout flexible de distribution de fluide (20) ; iii) un obturateur (25, 138) s'étendant dans ledit embout flexible (20) comprenant des côtés intérieur et extérieur, moyennant quoi ledit embout flexible (20) entre en contact hermétique avec une surface extérieure dudit obturateur (25, 138) lorsque la pression est égale sur les côtés intérieur et extérieur de l'embout flexible (20), et s'agrandit de façon radiale pour s'éloigner de ladite surface extérieure dudit obturateur (25, 138) lorsque le liquide sous pression est introduit dans un espace entre ledit obturateur (25, 138) et ledit embout flexible (20) ; iv) une chambre intermédiaire (136) communiquant avec ledit espace entre ledit obturateur (25, 138) et ledit embout flexible (20) et communiquant en alternance avec et isolée de façon hermétique de ladite chambre de stockage (122) ; et v) des moyens de soupape (40, 140) actionnables pour réguler l'écoulement dudit liquide stérile sous pression de ladite chambre de stockage (122) à ladite chambre intermédiaire (136) pour ainsi introduire sélectivement le liquide sous pression dans l'espace entre ledit obturateur (25, 138) et ledit embout flexible (20) et faire en sorte que ledit embout flexible (20) s'agrandisse pour s'éloigner de ladite surface extérieure dudit obturateur (25, 138) pour permettre au liquide d'être distribué à partir dudit embout flexible (20) ;
b) fournir un liquide stérile sous pression dans ladite chambre de stockage (122) ;
c) retourner ledit distributeur ;
d) appuyer sur ladite surface extérieure de ladite buse flexible (50, 126) pour ainsi actionner lesdits moyens de soupape (40, 140) et permettre au liquide stérile d'être distribué à partir dudit embout flexible (20) sous forme d'une ou de plusieurs gouttes ; et
e) relâcher la pression de la surface de ladite buse flexible (50, 126) lorsque le volume souhaité de liquide a été distribué.
